# FASCICULE DE BREVET EUROPEEN

(11) **EP 2 550 015 B1**
(45) Date de publication et mention de la délivrance du brevet: **22.02.2017**
(21) Numéro de dépôt: 11713007.0
(22) Date de dépôt: 15.03.2011
(51) Int. Cl.: A61K 39/00, A61K 39/12, A61K 39/215, A61K 39/17, A61K 39/39

(54) **VACCIN VIVANT POUR MALADIES AVIAIRES**
LEBENDIMPFSTOFFE GEGEN VOGELKRANKHEITEN
LIVE VACCINE FOR AVIARY DISEASES

(30) Priorité: 24.03.2010 FR 1052113
(43) Date de publication de la demande: 30.01.2013
(73) Titulaire: Société d'Exploitation de Produits pour les Industries Chimiques SEPPIC, 75007 Paris (FR)
(72) Inventeur: BERTRAND, François, 75014 Paris (FR); DEVILLE, Sébastien, 75010 Paris (FR); DUPUIS, Laurent, 51100 Reims (FR)
(74) Mandataire: Grout de Beaufort, François-Xavier
(86) Numéro de dépôt international: PCT/FR2011/050515
(87) Numéro de publication internationale: WO 2011/117507

(56) Documents cités:
- WO-A2-2005/009462
- WO-A2-2009/053601
- US-A- 4 650 677
- US-A- 4 795 635

## Description

La présente invention concerne un procédé de préparation d'une composition vaccinale, mise en oeuvre dans un traitement par administration par voie locale, de maladies virales aviaires et en particulier de la bronchite infectieuse aviaire.

La bronchite infectieuse aviaire est une maladie aiguë et contagieuse qui affecte les volailles principalement par des symptômes respiratoires. Le virus de la Bronchite Infectieuse Aviaire (ou VBI) est un membre du genre *Coronavirus,* famille des *Coronaviridae,* de l'ordre *Nidovirales.* Ce virus, à ARN simple brin possède une forte capacité d'évolution, et les particules virales peuvent survivre pendant une durée d'un mois dans le milieu extérieur.

Le VBI affecte les volailles de tous les âges et ne cible pas uniquement les voies respiratoires. En effet, le VBI se réplique tout d'abord dans la trachée et peut ensuite essaimer dans tout le corps de la volaille atteinte, en touchant différents organes internes.

La morbidité est élevée et la mortalité peut varier en fonction des élevages, des conditions d'élevages et aussi des terrains de surinfections bactériennes.

Les signes cliniques sont d'ordre :
- respiratoire (toux, râles trachéaux, sinus enflés, conjonctivite, ...) ;
- reproducteurs (diminution de la ponte, production d'oeufs à coquilles minces, déformées ou colorées) ;
- rénaux : soif intense...

Le VBI se transmet surtout par la voie respiratoire, ou par les aérosols. Les matières virulentes sont constituées par le jetage et les fientes. La transmission est horizontale, de façon directe (d'oiseaux malades à oiseaux sensibles), ou indirecte (par l'eau, le matériel,...).
Le VBI est sensible à la plupart des désinfectants mais le meilleur moyen de contention consiste en une vaccination alliée à la prise de mesure de bio-sécurité (mesures de décontamination, de désinfection, ...).

La vaccination consiste à inoculer à l'espèce à protéger une quantité de pathogène tué (vaccin inactivé) ou dont la virulence a été diminuée (vaccin vivant atténué) afin de déclencher une réponse biologique chez l'hôte, le protégeant lors de la survenue ultérieure de la maladie.
Les vaccins vivants sont généralement suffisamment efficaces pour ne pas nécessiter l'utilisation d'adjuvants.

Un adjuvant de vaccin est un excipient amplifiant la réponse biologique contre l'antigène auquel il est associé. On citera par exemple l'hydroxyde d'aluminium, les adjuvants huileux commercialisés sous l'appellation Montanide™ par la société SEPPIC. Ces adjuvants sont de natures diverses. Ils peuvent aussi bien consister en des liposomes, en des émulsions comprenant au moins une phase huile et au moins une phase aqueuse, du type adjuvants dits de Freund ou, de manière plus courante en des sels minéraux insolubles dans l'eau. Parmi les sels minéraux utilisés comme adjuvants de compositions vaccinales, on peut citer par exemple l'hydroxyde d'aluminium, le nitrate de cérium, le sulfate de zinc, l'hydroxyde de fer colloïdal ou le chlorure de calcium. L'hydroxyde d'aluminium est l'adjuvant le plus couramment utilisé. Ces sels minéraux utilisés comme adjuvants de compositions vaccinales sont décrits notamment dans l'article de Rajesh K. Gupta et al "Adjuvants, balance between toxicity and adjuvanticity", Vaccine, vol. 11, Issue 3, 1993, pages 993-306.

Les vaccins peuvent être administrés par voie injectable (injection sous cutanée ou intramusculaire) ou par voie locale (vaccination de masse par nébulisation, ajout dans l'eau de boisson pour contact avec le bec et les narines et le complexe occulonasal par exemple).
La vaccination par injection, même si elle s'avère efficace, présente comme inconvénient de ne pas être adaptée au contexte économique des élevages de volailles (coût de main d'oeuvre important), et elle provoque également un stress chez la volaille vaccinée, qui dans le cas des espèces pondeuses peut entraîner des perturbations dans la ponte suite au traumatisme physique généré par l'injection.
L'utilisation d'adjuvants efficaces dans les compositions vaccinales, et dans les compositions vaccinales destinées à prévenir la survenue de la bronchite infectieuse permet :
- d'augmenter l'intensité de la réponse protectrice permettant d'assurer un meilleur niveau de protection ;
- de prolonger la durée de la protection conférée par une dose de vaccin, assurant une protection plus durable des animaux dans les élevages tout au long de leur croissance ;
- d'assurer une protection suffisante avec un seul traitement quand deux traitements étaient nécessaires en absence de ces amplificateurs de réponse immunitaire. Le gain est alors dans le nombre de doses à injecter (divisé par deux), la manipulation des animaux (main d'oeuvre) et le stress généré lors de la manipulation des animaux (divisé aussi par deux) ;
- la possibilité d'obtenir, avec une dose antigénique moindre, une efficacité équivalente à celle conférée par une dose complète utilisée sans adjuvant. Ainsi, une entité de production de vaccin se verra, avec une même capacité productive, capable de produire un nombre supérieur de doses de vaccins. De même, un conditionnement existant pourra être proposé pour vacciner un plus grand nombre d'animaux.

Aucun adjuvant n'est décrit ou utilisé à ce jour lors de la mise en oeuvre des techniques de vaccinations par administration par voies locales utilisant des vaccins vivants.

Il existe un besoin de développer des diluants qui possèdent également la fonction adjuvante pour améliorer la réponse immunitaire telle que décrite ci-dessus. On nomme ces compositions des Diluants Adjuvants (DA).

La difficulté principale rencontrée dans le développement d'un DA est la faculté dudit diluant adjuvant (DA) à préserver vivant le vaccin vivant pour qu'il conserve ses propriétés immunogènes.

Un élément important du développement d'adjuvants pour les vaccins vivants réside dans la spécificité des formulations adjuvantes à ne pas tuer les micro-organismes vivants constituant les antigènes vaccinaux lors de leur mise en contact avant injection. Des DA existent sur le marché (comme par exemple l'acétate de tocophérol de la société Intervet compris dans le Diluvac Forte®) et sont recommandés pour certains vaccins vivants.

De plus un vaccin vivant est connu dans la demande de brevet français FR 2385401.

Toutefois, ce vaccin vivant n'est pas préparé avec des substances adjuvantes. Il a jusqu'ici été tenu pour acquis que la réponse immunitaire plus importante pourrait être réalisée avec un vaccin vivant, par exemple, en augmentant la teneur en virus, ou en utilisant une souche plus immunogène.

US 4 795 635 divulgue un procédé de préparation d'un vaccin comprenant un virus de la maladie de Newcastle.

WO 2005/009462 divulgue un procédé de préparation d'un vaccin comprenant un virus de la bronchite infectieuse.

WO 2009/053601 divulgue un vaccin démontrant des effets adjuvants améliorés tant sur la réponse humorale que cellulaire.

Selon la présente invention une méthode de préparation d'un vaccin à virus vivant a été trouvée, qui est caractérisée en ce que le vaccin vivant est préparé par le biais d'un adjuvant du type émulsion à phase continue aqueuse, par exemple une émulsion ou micro émulsion eau dans huile.

Il a étonnamment été constaté que l'utilisation d'une émulsion huile dans eau (H / E) comme "diluant" pour vaccins vivants a un effet positif sur la réponse sérologique et immunitaire chez les animaux vaccinés. Dans cette émulsion huile-dans-l'eau, la phase aqueuse externe permet au vaccin (en général lyophilisé) d'être facilement dissous.

Un autre aspect de l'invention est que l'utilisation de l'émulsion huile dans eau comme diluant adjuvant pour les vaccins vivants provoque une réponse sérologique très élevée chez les jeunes animaux ayant encore l'immunité maternelle. Cet effet surprenant peut être causé par l'action protectrice de l'émulsion sur le virus vivant contre la neutralisation par les anticorps présents chez l'animal.

Un but de la présente invention est de pallier tout ou partie des inconvénients de l'art antérieur relevés ci-dessus.

A cette fin, la présente invention a pour objet un procédé de préparation d'une composition vaccinale mise en oeuvre dans un traitement par administration par voie locale, d'une maladie virale aviaire comprenant au moins l'étape :
a) mélange d'un vaccin comprenant au moins un virus vivant choisi parmi un virus appartenant à une ou plusieurs souches de ladite maladie aviaire, extemporanément avec un diluant adjuvant (DA) ; caractérisé en ce que ledit diluant adjuvant est une micro-émulsion de type huile dans eau comprenant pour 100% de sa masse :
   - de 50% à 97% d'eau, plus particulièrement de 70% à 97% d'eau, et encore plus particulièrement de 80% à 97% d'eau ;
   - de 1% à 45% d'adjuvant huileux, plus particulièrement de 1% à 30% d'adjuvant huileux, et encore plus particulièrement de 2% à 10% d'adjuvant huileux ;
   - de 0,1% à 10% d'au moins un sel inorganique divalent, plus particulièrement de 0,1% à 8%, et encore plus particulièrement de 0,2% à 3% ;
   - de 0,1% à 10% d'au moins un agent complexant, plus particulièrement de 0,1% à 8%, et encore plus particulièrement de 0,2% à 3% ;
le dit au moins un agent complexant est choisi parmi l'acide éthylène diamine tetra acétique (EDTA), le gluconate de sodium, le gluconate de potassium et le polyacrylate de sodium.

Par maladie virale aviaire, on entend :
- les maladies virales respiratoires, comme par exemple la laryngotrachéite infectieuse, la bronchite infectieuse aviaire, l'herpesvirose du pigeon, la rhinotrachéite du dindon ;
- les maladies virales digestives, comme par exemple la peste du canard, l'hépatite virale du canard, les hepadnavirus aviaires ;
- les maladies virales de la peau, comme par exemple la variole aviaire ;
- les maladies virales du système nerveux comme par exemple l'encephalomyelite aviaire, l'encéphalite équine orientale, la fièvre du Nil Occidential, la rage, la « louping ill » ;
- les maladies virales généralisées comme par exemple la pseudo-peste aviaire (maladie de Newcastle), la maladie de Marek, la maladie de Gumboro, la peste aviaire, la leucose aviaire, l'anémie infectieuse du poulet, la maladie de Derszy, la maladie des « oeufs mous ».

Par diluant on entend une substance à ajouter à une autre pour diminuer son titre, sa richesse, ou son pourcentage.
Un Diluant Adjuvant pour Vaccin administré par voie locale (DAVAVL) devra, pour être pertinent :
- maintenir le vaccin vivant après reconstitution pendant une durée donnée ;
- permettre la préparation d'une formule (composition vaccinale) facilement pulvérisable (« sprayable ») et mouillante pour les muqueuses (de nature hydrophile) pour permettre un traitement efficace et facile ;
- améliorer significativement l'efficacité immunitaire de l'antigène par rapport à celle de l'antigène utilisé sans adjuvant, et sans générer d'effets secondaires.

Par ailleurs, des modes de réalisation de l'invention peuvent comporter l'une ou plusieurs des caractéristiques suivantes :
- procédé tel que défini ci-dessus, caractérisé en ce que ledit virus contenu dans ledit vaccin appartient à une ou plusieurs souches de VBI (Virus de la Bronchite Infectieuse) ;
- procédé tel que défini ci-dessus, caractérisé en ce que ledit virus contenu dans ledit vaccin est un virus de la maladie de Newcastle ;
- procédé tel que défini ci-dessus, caractérisé en ce que ledit virus contenu dans ledit vaccin appartient à une ou plusieurs souches de VBI (virus de la bronchite infectieuse), un virus de la maladie de Newcastle, ou un mélange de ces virus.
La maladie de Newcastle, aussi appelée « pseudopeste aviaire », « pneumoencéphalite aviaire » ou « maladie de Ranikhet », est une zoonose des oiseaux, due à un virus.

La morbidité et la mortalité varient fortement selon la virulence de la souche, l'immunité et l'état de l'animal et d'autres facteurs environnementaux.

Le virus de la maladie de Newcastle est un virus de la famille des Paramyxoviridés, du genre Rubulavirus.

C'est un virus ARN, à un seul brin (à la différence de la grippe qui a huit brins), dit monocaténaire. Les virus à ARN mutent facilement et souvent, ce qui peut rendre les stratégies pharmaceutiques et vaccinales plus complexes et difficiles.

L'enveloppe d'un diamètre de 150 à 300 nm présente deux types de spicules glycoprotéiniques. Elle est caractérisée par :
- Une glycoprotéine HN, possédant les activités hémagglutinante et neuramidasique, permettant l'attachement du virion sur des récepteurs membranaires à la surface de la cellule et son relarguage ;
- Une glycoprotéine F qui permet la fusion entre l'enveloppe virale et la membrane cellulaire.
La culture du virus se fait aisément dans des oeufs de poule embryonnés ou *in vitro* (sur fibroblastes d'embryons de poulet ou sur des cellules rénales de poulet).
Le virus est très résistant à température ambiante, il reste infectieux :
- longtemps (plusieurs mois) dans les matières fécales,
- 2 à 3 mois au sol, dans un poulailler,
- 7 à 8 mois sur une coquille souillée,
- 2 ans et plus dans une carcasse non cuite et congelée.
Comme pour la grippe on classe les souches selon leur virulence en distinguant :
- des souches vélogènes (très virulentes, induisant une mortalité approchant ou atteignant les 100 %, avec une attaque systémique, ou au moins viscérale ou nerveuse associée ou non à des troubles respiratoires),
- des souches mésogènes (moyennement virulentes) produisant une affection respiratoire avec troubles nerveux pour une mortalité atteignant 50% chez les jeunes oiseaux,
- des souches lentogènes (faiblement virulentes, non mortelles, produisant quelques troubles respiratoires, et parfois n'induisant aucun symptôme). Ce sont par exemple les souches Hitchner B1 et La Sota.
Les sources du virus sont liées aux organes ciblés par le virus, qui varient selon la souche virale, l'état et l'histoire immunitaire de l'oiseau touché qui exprimera le virus dans :
- les sécrétions bronchiques et matières fécales,
- toutes les parties de la carcasse.

Les virus sont excrétés dès l'incubation et sur une période variable lors de la convalescence, quelques jours à deux semaines, rarement plus.

Pour lutter contre le virus, il existe des mesures de précaution et de prévention qui peuvent être mises en oeuvre par l'éleveur, qui peuvent par exemple consister en une mise en quarantaine des animaux infectés, en une euthanasie des animaux infectés, de mesures d'hygiène, en une désinfection et un nettoyage régulier des locaux avec des désinfectants conventionnels.

La vaccination constitue aussi un moyen de prévention.

L'invention a également pour objet :
- Un procédé tel que défini ci-dessus, caractérisé en ce que le virus contenu dans ledit vaccin vivant est lyophilisé avant l'étape a).
Les vaccins vivants sont généralement conservés lyophilisés et doivent être remis en suspension de façon extemporanée avec une phase aqueuse. Le vaccin ainsi reconstitué devant être utilisé dans les heures qui suivent l'ajout d'un diluant.
- Un procédé tel que défini ci-dessus, caractérisé en ce que ledit adjuvant huileux comprend pour 100% de sa masse :
   - de 40% à 95% d'au moins une huile minérale, plus particulièrement de 50% à 95% d'au moins une huile minérale, et encore plus particulièrement de 50% à 90% d'au moins une huile minérale ;
   - de 5% à 60% d'au moins un tensioactif, plus particulièrement de 5% à 50% d'au moins un tensioactif, et encore plus particulièrement de 10% à 50% d'au moins un tensioactif.
- Un procédé tel que défini ci-dessus, caractérisé en ce que ledit au moins un sel inorganique divalent est choisi parmi le gluconate de manganèse, le gluconate de calcium, l'aspartate de calcium, le gluconate de zinc, le gluconate de fer, le chlorure de calcium.

Les huiles minérales utilisées pour la préparation des adjuvants huileux sont sélectionnées dans le groupe constitué par les huiles minérales, hydrocarbures, obtenues par distillation du pétrole et par la mise en oeuvre d'étapes de traitement subséquentes comme par exemple les étapes de désulfurisation, de désasphaltage, d'extraction des composés aromatiques, d'extraction des cires, et autres étapes de traitement de finition (on peut citer par exemple les huiles du type MARCOL 52, MARCOL 82, DRAKEOL 5 ET DRAKEOL 6, ...).

Les tensioactifs présents dans les adjuvants huileux sont des tensioactifs émulgateurs, présentant un caractère hydrophile caractérisé par une valeur HLB comprise entre 8 et 19, plus particulièrement comprise entre 8 et 15.

Un tel tensioactif peut consister en un alkylpolyglycoside ou un mélange d'alkylpolyglycosides ; des saponines ; des lécithines ; des alcanols polyoxyéthylés ; des polymères comprenant des blocs polyoxyéthylènes et polyoxypropylènes ; des esters obtenus par condensation d'un acide gras, avantageusement un acide gras liquide à 20°C avec un sucre, du sorbitol, du mannitol ou du glycérol. Ledit sucre peut consister en du glucose ou du saccharose ou, de préférence, mannitol. A titre d'esters préférés, on peut citer les esters d'acides gras, comme par exemple l'acide oléique, l'acide stéarique, l'acide palmitique, l'acide laurique, et de sorbitol ou de mannitol, obtenus par estérification de l'acide gras avec le sorbitol ou le mannitol, ou bien par estérification avec les produits résultant de l'anhydrisation de la chaîne polyhydroxylée du sorbitol ou du mannitol qui se cyclise en position 1-4 ou en position 2-6, ou bien par estérification avec le sorbitol ou le mannitol et avec les produits résultant de l'anhydrisation de la chaîne polyhydroxylée du sorbitol ou du mannitol qui se cyclise en position 1-4 ou en position 2-6. Comme esters de mannitol particulièrement préférés, on peut citer les oléates de mannitol, les oléates de mannitan, les oléates de mannitol éthoxylés à 5 moles ou 10 moles ou 15 moles ou 20 moles d'oxyde d'éthylène, les oléates de mannitan éthoxylés à 5 moles ou 10 moles ou 15 moles ou 20 moles d'oxyde d'éthylène. Des dérivés d'esters de sucre de polyéthylèneglycol, de sorbitol ou de glycérol peuvent être également mis en oeuvre. Les autres types de tensioactifs préférés consistent en des huiles végétales éthoxylées, comme par exemple des huiles de maïs éthoxylées ayant entre 3 moles et 40 moles d'oxyde d'éthylène, des huiles de colza éthoxylées ayant entre 3 moles et 40 moles d'oxyde d'éthylène, des huiles de ricin éthoxylées ayant entre 3 moles et 60 moles d'oxyde d'éthylène.

### EXEMPLES

La compatibilité des formules adjuvantes avec la viabilité des vaccins lyophilisés est liée à la composition de cette formule adjuvante et à son taux d'utilisation. La sélection de constituants biocompatibles combinés dans des proportions assurant une bonne mise en oeuvre et un pouvoir adjuvant a été effectuée puis évaluée dans des protocoles d'études quantitatives. La viabilité des vaccins adjuvés (et donc la compatibilité des formulations avec les vaccins vivants) est forcement bonne pour les vaccins ayant démontré une efficacité supérieure à une référence commerciale.

L'amélioration de l'efficacité se traduit pour les éleveurs par de meilleures performances zootechniques, un moindre coût pour les traitements et un gain de temps. Les traitements par voie locale n'utilisent aujourd'hui pas d'adjuvants stimulateurs de performances.

### A- Mise en évidence dans le cas de la Bronchite Infectieuse.

### a) Produits objets de l'étude.

### Différents Diluants Adjuvants préparés.

**Tableau I**

| | DA1 | DA2 | DA3 | DA4 | DA5 | DA6 | DA7 | DA8 | DA9 | DA10 | DA11 |
|---|---|---|---|---|---|---|---|---|---|---|---|
| Eau | 83% | 83% | 86% | 86% | 86% | 83% | 89% | 89% | 86% | 98% | 75% |
| Adjuvant : *Huile* | | | | | | | | | | | |
| Marcol 52 ; *Oléate de mannitan éthoxylé HLB = 12* | 5% | 5% | 5% | 5% | 5% | 5% | 5% | 5% | 5% | 0% | 15% |
| | 5% | 5% | 2% | 2% | 5% | 5% | 2% | 2% | 5% | 0% | 10% |
| Agent Complexant | | | | | | | | | | | |
| EDTA | 0%5% | 0% | 0% | 0% | 2% | 0% | 2% | 0% | 2% | 0% | 0% |
| PolyAcrylate de sodium | | 0% | 5% | 0% | 0% | 0% | 0% | 0% | 0% | 2% | 0% |
| Sel minéral | | | | | | | | | | | |
| - Gluconate de Manganèse | 2% | 2% | 2% | 2% | 2% | 2% | 2% | 2% | 0% | 0% | 0% |
| - Gluconate de Calcium | 0% | 0% | 0% | 0% | 0% | 0% | 0% | 0% | 2% | 0% | 0% |
| Forme physique du Diluant Adjuvant | mH/E | mH/E | H/E | H/E | m H/E | M H/E | H/E | H/E | m H/E | - | H/E |

| | | | | | | | | | | | |
|---|---|---|---|---|---|---|---|---|---|---|---|
| - m H/E : micro-émulsions H/E - H/E: émulsion H/E | | | | | | | | | | | |

### Différentes Compositions Vaccinales préparées.

Antigène utilisé : virus de la lignée H-120 du Virus B1 extrait d'oeufs embryonnés de poulets exempts de pathogènes (SPF) avec un titre d'infectivité effective de Ig 7.0 EID50/mL.

Chaque vaccin expérimental (de VA1 à VA11, comprenant respectivement le Diluant Adjuvant de DA1 à DA11) est préparé par ajout de :
- environ 5% de l'antigène (correspondant au lyophilisat de l'antigène décrit ci-dessus), et de
- environ 95% du Diluant Adjuvant expérimental (DA1 à DA11).

### b) Mesures d'efficacité sur le poulet.

### Protocole expérimental.

b1) L'efficacité de chacune des compositions vaccinales (VA1 à VA11) a été déterminée sur des groupes de 10 poulets recevant un traitement de 100 µl de vaccin dans chaque narine (administration intranasale de 0,2cm³ de vaccin par animal).

Ainsi, les poulets du groupe i reçoivent un traitement impliquant le vaccin VAi, avec i = 1 à 11. Les poulets utilisés dans le cadre de cette expérimentation sont issus d'une ferme, âgés de 22 jours, séronégatifs pour la maladie Bronchite Infectieuse BI, de la souche Highsex brunes.

D'autre part :
- un groupe de 10 poulets (Groupe 12), ou groupe témoin positif, reçoit l'administration selon le même mode d'un vaccin commercial contre la Bronchite Infectieuse. Ce vaccin commercial ne contient que de l'eau comme diluant et pas d'adjuvant, ni d'huile, ni de tensioactif.
   Comme référence positive nous avons utilisé un vaccin commercial produit par FGI « ARRIAH » : IB souche H-120 lot 211.
- un groupe de 10 poulets (Groupe 13), ou groupe témoin négatif, ne reçoit pas de vaccination.

Pour chacun des poulets de chaque groupe, des prélèvements sanguins ont été effectués 7/14/21/28/35/42/49/56 jours après le début de la vaccination pour quantification des titres anticorps générés.

### b2) Epreuve de virulence

56 jours après la date de la première vaccination, la protection générée par les vaccins est évaluée en effectuant une épreuve virulente : pour cela, on infecte les animaux avec une charge déterminée de pathogène (utilisant la souche pathogène M-41 du virus IB à une dose de 6.46 CD50 ou Ig 6.3 EID 50).

L'intensité de la maladie provoquée est évaluée en mesurant :
1/ l'intensité cumulée des points de chaque animal pendant 7 jours suivant l'infection avec une échelle de notation (1 point : respiration courte / 2 points : respiration courte et difficultés trachéales / 3 points : détresse respiratoire, face turgescente, écoulements, symptômes généraux). Plus un groupe sera malade longtemps et intensément et plus ce score sera élevé ;
2/ la somme des jours du groupe pendant lesquels les symptômes ont été observés : cette notation quantifie l'aptitude des animaux à vaincre la maladie ;
3/ la moyenne des points avec l'écart type du groupe permettant d'évaluer l'homogénéité du groupe ;
4/ le retard dans le déclanchement de la maladie : certains traitements retardent l'apparition des symptômes mais au détriment de la faculté à vaincre la maladie ;
5/ le taux d'animaux tombant malades.

Ces évaluations sont réalisées par un vétérinaire dûment qualifié et entraîné dansle cadre d'un protocole expérimental habituellement mis en oeuvre dans le but d'évaluer les performances de vaccins contre IB.

### Résultats expérimentaux.

### Titres anticorps :

L'ensemble des résultats est présenté dans le tableau II.

Le groupe d'animaux non vaccinés (groupe 13) ne développe pas d'anticorps significativement (environ 1 avec un écart type d'environ 0,2) ce qui signifie qu'il n'y a pas eu de contamination par le virus pendant l'essai, validant ainsi les autres résultats.

Le vaccin commercial (groupe 12) développe des titres supérieurs à 3 après 28 jours, avec un écart type d'environ 0,2 dès 14 jours après vaccination et stable durant les 56 jours.

Les groupes 1/3/5/7/9 développent des titres anticorps supérieurs à 3 (jusqu'à 4) et ceci à partir de 21 jours et pour toute la durée de l'essai avec des écart-types d'environ 0,2.

Ces groupes présentent des résultats significativement supérieurs par rapport au vaccin commercial (P noté dans le tableau).

Les autres groupes présentent des niveaux de réponse similaires ou inférieurs au vaccin commercial.

P définit la significativité des résultats. C'est à dire la probabilité d'occurrence qui permet d'établir si les différences sont significatives.
P<0.01 : différence significative par rapport au vaccin commercial.
P<0.001 : différence très significative par rapport au vaccin commercial.

**Tableau II : résultats de titres anticorps des groupes 1 à 13 avant épreuve virulente pendant 56 jours.**

| | Jours après vaccination | | | | | | | | |
|---|---|---|---|---|---|---|---|---|---|
| | Avant vaccination | 7 | 14 | 21 | 28 | 35 | 42 | 49 | 56 |
| Groupe 1 | 2.11 ±0.13 | 1.40 ±0.23 | 2.31 ±0.13 | 2.65 ±0.07 | 2.89 ±0.13 | 3.19 ±0.13 | 3.99 ±0.04 (P<0,001)** | 4.01 ±0.04 (P<0,001) | 3.76 ±0.08 (P<0,001) |
| Groupe 2 | 1.46 ±0.27 | 1.18 ±0.25 | 2.42 ±0.12 | 2.60 ±0.14 | 3.22 ±0.17 | 3.23 ±0.14 | 3.21±0.15 | 3.24 ±0.13 | 3.15 ±0.16 |
| Groupe 3 | 2.11 ±0.13 | 1.40 ±0.23 | 2.31 ±0.13 | 2.65 ±0.07 | 2.89 ±0.13 | 3.19 ±0.13 | 3.99 ±0.04 (P<0,001)** | 4.01 ±0.04 (P<0,001) | 3.76 ±0.08 (P<0,001) |
| Groupe 4 | 2.08 ±0.23 | 1.39 ±0.29 | 2.48 ±0.15 | 2.74 ±0.2 | 2.55 ±0.44 | 3.03 ±0.18 | 3.10 ±0.14 | 3.11 ±0.19 | 3.68±0.11 (P<0,001) |
| Groupe 5 | 1.68 ±0.32 | 1.85 ±0.22 | 2.61 ±0.17 | 3.24 ±0.17 | 3.64 ±0.10 (P≤0,01) | 3.72 ±0.10 (P<0,02) | 3.70 ±0.11 (P<0,001) | 3.48 ±0.14 | 3.22 ±0.16 |
| Groupe 6 | 1.40 ±0.27 | 0.68 ±0.25 | 2.55 ±0.18 | 2.97 ±0.21 | 2.90 ±0.20 | 3.05 ±0.12 | 3.01 ±0.13 | 2.92 ±0.15 | 2.82 ±0.17 |
| Groupe 7 | 1.91 ±0.29 | 1.44 ±0.22 | 2.84 ±0.14 | 2.90 ±0.17 | 3.24 ±0.20 | 3.90 ±0.05 (P<0,001) | 3.90 ±0.05 (P<0,001) | 3.69 ±0.08 (P<0,01) | 3.46±0.1 (P<0,01) |
| Groupe 8 | 1.60 ±0.25 | 1.91 ±0.14 | 2.77 ±0.19 | 3.11 ±0.22 | 3.28 ±0.21 | 3.38 ±0.17 | 3.23 ±0.19 (P<0,01) | 3.20 ±0.14 | 3.06 ±0.17 |
| Groupe 9 | 1.62 ±0.22 | 1.75 ±0.20 | 2.81 ±0.23 | 3.43 ±0.16 | 3.74 ±0.32 (P≤0,01) | 3.82 ±0.15 (P<0,02) | 3.75 ±0.19 (P<0,001) | 3.48 ±0.14 | 3.22 ±0.16 |
| Groupe 10 | 1.97 ±0.10 | 1.33 ±0.18 | 2.62 ±0.12 | 2.91 ±0.12 | 3.14 ±0.11 | 3.09±0.15 | 3.08±0.14 | 2.85 ±0.13 | 2.63 ±0.14 |
| Groupe 11 | 1.11 ±0.13 | 1.54 ±0.08 | 2.32 ±0.23 | 3.01 ±0.11 | 3.04 ±0.21 | 3.11 ±0.12 | 3.12 ±0.19 | 2.52 ±0.23 | 2.53 ±0.54 |
| Groupe 12 Vaccin commercial | 1.72 ±0.21 | 1.42 ±0.29 | 2.58 ±0.18 | 2.81 ±0.17 | 3.15 ±0.14 | 3.27 ±0.13 | 3.09 ±0.10 | 3.11 ±0.15 | 3.07 ±0.04 |
| Groupe 13 Animaux non traits | 1.65 ±0.26 | 0.50 ±0.21 | 1.00 ±0.22 | 0.44 ±0.23 | 0.79 ±0.20 | 0.80 ±0.21 | 0.76 ±0.18 | 0.58 ±0.24 | 0.38 ±0.21 |

### Épreuve de virulence:

**Tableau III : résultats de comportement à l'épreuve virulente des groupes 1 à 13 pour les scores de résistance au virus.**

| Groupe | Nombre de poulets | Points cumulés | Jours cumulés de symptômes cliniques | Moyenne de points / poulet ± écart type | Retard moyen de début symptômes ± écart type | Proportion déclenchant la maladie |
|---|---|---|---|---|---|---|
| 1 | 10 | 2 | 2 | 0,20±0,38 | 0,20±0,42 | 0,40 |
| 2 | 10 | 32 | 11 | 3,2±0,71 | 3,39±2,84 | 0,80 |
| 3 | 10 | 2 | 3 | 0,20±0,11 | 0,20±0,32 | 0,10 |
| 4 | 10 | 34 | 33 | 3,40±2,80 | 3,30±2,75 | 0,90 |
| 5 | 10 | 5 | 5 | 0,50±0,70 | 1,00±0,71 | 0,30 |
| 6 | 10 | 13 | 12 | 1,30±0,28 | 1,90±0,88 | 0,60 |
| 7 | 10 | 3 | 3 | 0,30±0,48 | 0,30±0,48 | 0,30 |
| 8 | 10 | 12 | 11 | 1,20±0,88 | 1,10±0,88 | 0,70 |
| 9 | 10 | 2 | 2 | 0,20±0,42 | 0,20±0,42 | 0,30 |
| 10 | 10 | 12 | 11 | 1,20±1,23 | 1,10±1,29 | 0,70 |
| 11 | 10 | 8 | 8 | 0,80±1,03 | 0,80±1,03 | 0,50 |
| 12 Vaccin commerci al | 9 | 22 | 21 | 2,44±1,94 | 2,33±1,73 | 0,50 |
| 13 Control négatif | 12 | 109 | 83 | 9,08±1,83 | 6,92±1,44 | 1,00 |

Le groupe témoin non traité (Groupe 13) développe des scores d'intensité de symptômes très élevés (environ 9 par poulet, pour 100% des animaux, avec beaucoup de jours de maladie, et un retard dans le déclenchement des symptômes de la maladie d'environ 7 jours). Ces résultats valident l'épreuve virulente.

L'utilisation du vaccin commercial (Groupe 12) réduit significativement tous ces paramètres en maintenant cependant 50% des poulets qui développent la maladie, après seulement deux jours de retard, mais avec des symptômes modérés (22 points cumulés) pendant une relativement courte période (seulement 21 jours cumulés).

Les groupes 1/3/5/7/9 montrent des scores de points cumulés très faibles (0,2 à 0,5) pour des durées brèves (2 à 5 jours cumulés) sans retarder significativement les symptômes de la maladie et en protégeant dans chaque groupe au moins 60% de la population.

Les autres groupes donnent des scores intermédiaires entre le vaccin commercial et les groupes 1/3/5/7/9 mais sans induire de protection supérieure à 50% de la population.

## Revendications

1. Procédé de préparation d'une composition vaccinale mise en oeuvre dans un traitement par administration par voie locale, d'une maladie virale aviaire comprenant au moins l'étape :
a) mélange d'un vaccin comprenant au moins un virus vivant choisi parmi un virus appartenant à une ou plusieurs souches de ladite maladie aviaire, extemporanément avec un diluant adjuvant (DA) ; **caractérisé en ce que** ledit diluant adjuvant est une micro-émulsion de type huile dans eau comprenant pour 100% de sa masse :
- de 50% à 97% d'eau, plus particulièrement de 70% à 97% d'eau, et encore plus particulièrement de 80% à 97% d'eau ;
- de 1% à 45% d'adjuvant huileux, plus particulièrement de 1% à 30% d'adjuvant huileux, et encore plus particulièrement de 2% à 10% d'adjuvant huileux ;
- de 0,1% à 10% d'au moins un sel inorganique divalent, plus particulièrement de 0,1% à 8%, et encore plus particulièrement de 0,2% à 3% ;
- de 0,1% à 10% d'au moins un agent complexant, plus particulièrement de 0,1% à 8%, et encore plus particulièrement de 0,2% à 3% ;
le dit au moins un agent complexant est choisi parmi l'acide éthylène diamine tetra acétique (EDTA), le gluconate de sodium, le gluconate de potassium et le polyacrylate de sodium.

2. Procédé selon la revendication précédente **caractérisé en ce que** ledit virus contenu dans ledit vaccin appartient à une ou plusieurs souches de VBI (Virus de la Bronchite Infectieuse).

3. Procédé selon la revendication 1 **caractérisé en ce que** ledit virus contenu dans ledit vaccin est un virus de la maladie de Newcastle.

4. Procédé selon la revendication 1 **caractérisé en ce que** ledit virus contenu dans ledit vaccin appartient à une ou plusieurs souches de VBI (virus de la bronchite infectieuse), un virus de la maladie de Newcastle, ou un mélange de ces virus.

5. Procédé selon l'une des revendications précédentes **caractérisé en ce que** le virus contenu dans ledit vaccin vivant est lyophilisé avant l'étape a).

6. Procédé selon l'une des revendications 1 à 5, **caractérisé en ce que** ledit adjuvant huileux comprend pour 100% de sa masse :
- de 40% à 95% d'au moins une huile minérale, plus particulièrement de 50% à 95% d'au moins une huile minérale, et encore plus particulièrement de 50% à 90% d'au moins une huile minérale ;
- de 5% à 60% d'au moins un tensioactif, plus particulièrement de 5% à 50% d'au moins un tensioactif, et encore plus particulièrement de 10% à 50% d'au moins un tensioactif.

7. Procédé selon l'une des revendications 1 à 6 **caractérisé en ce que** ledit au moins un sel inorganique divalent est choisi parmi le gluconate de manganèse, le gluconate de calcium, l'aspartate de calcium, le gluconate de zinc, le gluconate de fer, le chlorure de calcium.

## Patentansprüche

1. Verfahren zur Herstellung einer Impfstoffzusammensetzung, die in einer Behandlung einer viralen Vogelkrankheit mittels lokaler Verabreichung eingesetzt wird, welches mindestens den Schritt umfasst:
a) Vermischen eines Impfstoffs, welcher mindestens ein Lebendvirus umfasst, ausgewählt aus einem Virus, das einem oder mehreren Stämmen der Vogelkrankheit angehört, kurz vor Gebrauch mit einem Verdünnungs-Hilfsstoff (VH); **dadurch gekennzeichnet, dass** es sich bei dem Verdünnungs-Hilfsstoff um eine Mikroemulsion des Typs Öl-in-Wasser handelt, welche auf 100 % seiner Masse Folgendes umfasst:
- 50 % bis 97 % Wasser, spezieller 70 % bis 97 % Wasser, und noch spezieller 80 % bis 97 % Wasser;
- 1 % bis 45 % öligen Hilfsstoff, spezieller 1 % bis 30 % öligen Hilfsstoff, und noch spezieller 2 % bis 10 % öligen Hilfsstoff;
- 0,1 % bis 10 % mindestens eines zweiwertigen anorganischen Salzes, spezieller 0,1 % bis 8 %, und noch spezieller 0,2 % bis 3 %;
- 0,1 % bis 10 % mindestens eines Komplexbildners, spezieller 0,1 % bis 8 %, und noch spezieller 0,2 % bis 3 %;
wobei der mindestens eine Komplexbildner ausgewählt ist aus Ethylendiamintetraessigsäure (EDTA), Natriumgluconat, Kaliumgluconat und Natriumpolyacrylat.

2. Verfahren nach dem vorstehenden Anspruch, **dadurch gekennzeichnet, dass** das Virus, welches in dem Impfstoff enthalten ist, einem oder mehreren Stämmen des IBV (infektiöses Bronchitisvirus) angehört.

3. Verfahren nach Anspruch 1, **dadurch gekennzeichnet, dass** es sich bei dem Virus, welches in dem Impfstoff enthalten ist, um ein Virus der Newcastle-Krankheit handelt.

4. Verfahren nach Anspruch 1, **dadurch gekennzeichnet, dass** das Virus, welches in dem Impfstoff enthalten ist, einem oder mehreren Stämmen des IBV (infektiöses Bronchitisvirus), einem Virus der Newcastle-Krankheit, oder einer Mischung dieser Viren angehört.

5. Verfahren nach einem der vorstehenden Ansprüche, **dadurch gekennzeichnet, dass** das Virus, welches in dem Lebendimpfstoff enthalten ist, vor Schritt a) gefriergetrocknet wird.

6. Verfahren nach einem der Ansprüche 1 bis 5, **dadurch gekennzeichnet, dass** der ölige Hilfsstoff auf 100 % seiner Masse Folgendes umfasst:
- 40 % bis 95 % mindestens eines Mineralöls, spezieller 50 % bis 95 % mindestens eines Mineralöls, und noch spezieller 50 % bis 90 % mindestens eines Mineralöls;
- 5 % bis 60 % mindestens eines Tensids, spezieller 5 % bis 50 % mindestens eines Tensids, und noch spezieller 10 % bis 50 % mindestens eines Tensids.

7. Verfahren nach einem der Ansprüche 1 bis 6, **dadurch gekennzeichnet, dass** das mindestens eine zweiwertige anorganische Salz ausgewählt ist aus Mangangluconat, Calciumgluconat, Calciumaspartat, Zinkgluconat, Eisengluconat, Calciumchlorid.

## Claims

1. Method for preparing a vaccine composition used in a treatment, administered locally, for an avian viral disease, comprising at least the step:
a) extemporaneously mixing a vaccine comprising at least one live virus chosen from a virus belonging to one or more strains of said avian disease with an adjuvant diluent (AD), **characterised in that** said adjuvant diluent is an oil-in-water microemulsion comprising, for 100% of the mass thereof:
- 50% to 97% water, more particularly 70% to 97% water, and even more particularly 80% to 97% water;
- 1% to 45% oily adjuvant, more particularly 1% to 30% oily adjuvant, and even more particularly 2% to 10% oily adjuvant;
- 0.1% to 10% of at least one divalent inorganic salt, more particularly 0.1% to 8%, and even more particularly 0.2% to 3%;
- 0.1% to 10% of at least one complexing agent, more particularly 0.1% to 8%, and even more particularly 0.2% to 3%;
said at least one complexing agent is chosen from ethylenediaminetetraacetic acid, sodium gluconate, potassium gluconate and sodium polyacrylate.

2. Method according to the previous claim, **characterised in that** said virus contained in said vaccine belongs to one or more strains of IBV (infectious bronchitis virus).

3. Method according to claim 1, **characterised in that** said virus contained in said vaccine is a Newcastle disease virus.

4. Method according to claim 1, **characterised in that** said virus contained in said vaccine belongs to one or more strains of IBV (infectious bronchitis virus), a Newcastle disease virus, or a mixture of these viruses.

5. Method according to one of the previous claims, **characterised in that** the virus contained in said live vaccine is freeze-dried before step a).

6. Method according to one of claims 1 to 5, **characterised in that** said oily adjuvant comprises, for 100% of the mass thereof:
- 40% to 95% of at least one mineral oil, more particularly 50% to 95% of at least one mineral oil, and even more particularly 50% to 90% of at least one mineral oil;
- 5% to 60% of at least one surfactant, more particularly 5% to 50% of at least one surfactant, and even more particularly 10% to 50% of at least one surfactant.

7. Method according to one of claims 1 to 6, **characterised in that** said at least one divalent inorganic salt is chosen from manganese gluconate, calcium gluconate, calcium aspartate, zinc gluconate, iron gluconate and calcium chloride.
